# EUROPEAN PATENT SPECIFICATION

(11) **EP 3 823 609 B1**
(45) Date of publication and mention of the grant of the patent: **04.03.2026**
(21) Application number: 19838008.1
(22) Date of filing: 16.07.2019
(51) Int. Cl.: A61K 31/192, A61K 31/196, A61K 31/235, A61P 25/18, A61K 45/06

(54) **SODIUM BENZOATE FOR USE IN PREVENTING OR TREATING ANTI-N-METHYL-D-ASPARTATE RECEPTOR ENCEPHALITIS**
NATRIUMBENZOATE ZUR VERWENDUNG BEI DER VORBEUGUNG ODER BEHANDLUNG VON ANTI-N-METHYL-D-ASPARTATREZEPTOR-ENCEPHALITIS
BENZOATE SODIQUE POUR UNE UTILISATION DANS LA PRÉVENTION OU LE TRAITEMENT DE L'ENCÉPHALITE DU RÉCEPTEUR ANTI-N-METHYL-D-ASPARTATE

(30) Priority: 16.07.2018 US 201862698558 P
(43) Date of publication of application: 26.05.2021
(73) Proprietor: Excelsior Pharmatech Labs, Taipei City 115 (TW); Tzang, Ruu-Fen, Taipei (TW); Lane, Hsien-Yuan, Taichung City 404 (TW)
(72) Inventor: TZANG, Ruu-Fen, Taipei (TW); LANE, Hsien-Yuan, Taichung City 404 (TW)
(74) Representative: Icosa
(86) International application number: PCT/MY2019/000025
(87) International publication number: WO 2020/017950

(56) References cited:
- WO-A1-2014/191992
- DALMAU J ET AL: "Clinical experience and laboratory investigations in patients with anti-NMDAR encephalitis", THE LANCET NEUROLOGY, ELSEVIER, AMSTERDAM, NL, vol. 10, no. 1, 1 January 2011 (2011-01-01), pages 63 - 74, XP027599021, ISSN: 1474-4422, [retrieved on 20110101], DOI: 10.1016/S1474-4422(10)70253-2
- GUAN ET AL.: "D-cycloserin, a NMDA-agonist may be a treatment option for anti-NMDAR encephalitis", NEUROIMMUNOLOGY AND NEUROINFLAMMATION, vol. 3, 2016, pages 189 - 191, XP055680865
- HERESCO-LEVY ET AL.: "Clinical and Electrophysiological Effects of D-Serine in a Schizophrenia Patient Positive for Anti-N-Methyl-D-Aspartate Receptor Antibodies", BIOLOGICAL PSYCHIATRY, vol. 77, 15 March 2015 (2015-03-15), pages e27 - e29, XP029221131
- LANE ET AL.: "Add-on Treatment of Benzoate for Schizophrenia a Randomized, Double- blind, Placebo-Controlled Trial of D-Amino Acid Oxidase Inhibitor", JAMA PSYCHIATRY, vol. 70, no. 12, 2013, pages 1267 - 1275, XP055553505
- AKIKO MATSUURA , YUKO FUJITA , MASAOMI IYO , KENJI HASHIMOTO: "Effects of sodium benzoate on pre-pulse inhibition deficits and hyperlocomotion in mice after administration of phencyclidine", ACTA NEUROPSYCHIATRICA, vol. 27, no. 3, June 2015 (2015-06-01), pages 159 - 167, DOI: 10.1017/neu.2015.1
- HUGHES ET AL.: "Cellular and Synaptic Mechanisms of Anti-NMDA Receptor Encephalitis", THE JOURNAL OF NEUROSCIENCE, vol. 30, no. 17, 28 April 2010 (2010-04-28), pages 5866 - 5875, XP055680876
- TERRY-LORENZO ET AL.: "Novel human D-amino acid oxidase inhibitors stabilize an active- site lid-open conformation", BIOSCIENCE REPORTS, vol. 34, 2014, pages 487 - 499, XP055680881
- SERSHEN ET AL.: "Modulating NMDA Receptor Function with D-Amino Acid Oxidase Inhibitors: Understanding Functional Activity in PCP-Treated Mouse Model", NEUROCHEM RES., vol. 41, February 2016 (2016-02-01), pages 398 - 408, XP035925838

## Description

### BACKGROUND

### 1. Technical Field

The present disclosure relates to a pharmaceutical composition for use in preventing or treating anti-N-methyl-D-aspartate receptor (NMDAR) encephalitis in a subject in need thereof, and particularly to pharmaceutical compositions including an effective amount of sodium benzoate and a pharmaceutically acceptable excipient thereof for use in preventing or treating anti-NMDAR encephalitis.

### 2. Description of Associated Art

Anti-N-methyl-D-aspartate receptor (NMDAR) encephalitis is an autoimmune encephalitis caused by NMDAR dysfunction associated with autoantibodies. Its occurrence has been increasingly identified in the past decade. The autoantibodies pass the blood-brain barrier and selectively crosslink with NMDA receptors, resulting in internalization of NMDA receptors in both excitatory and inhibitory hippocampal neurons that eventually causes hypofunction of NMDAR-mediated synaptic neurotransmission.

Majority of patients affected with anti-NMDAR encephalitis revealed first the psychotic symptom, followed by neurologic symptom such as epilepsy. Usually, anti-epileptics and neuroleptic drugs are less effective in patients with anti-NMDAR encephalitis. In addition, therapies involving the removal of autoantibodies by immune therapy with methylprednisolone and intravenous immunoglobulin or by plasma exchange are also used to treat patients with anti-NMDAR encephalitis. However, some patients remain refractory even after immune therapy and antipsychotic treatment.

There are also some publications that tried to provide different solutions to the prophylaxis or treatment of anti-NMDAR encephalitis or other psychosis. For example, DALMAU J et al. (THE LANCET NEUROLOGY, ELSEVIER, AMSTERDAM, NL, vol. 10, no. 1, 1 January 2011, pages 63-74) mentions the use of immunotherapy or antipsychotic medication against anti-NMDAR encephalitis; WO 2014/191992 A1 discloses a method of treating anti-NMDAR encephalitis via the administration of a NMDAR agonist, an alanine-serine-cysteine transporter inhibitor, a D-amino acid oxidase inhibitor, a glycine transport inhibitor or a combination thereof; GUAN et al. (Neuroimmunology and Neuroinflammation, vol. 3, 2016, pages 189-191) discloses the use of D-cycloserine treatment of anti-NMDAR encephalitis; HERESCO-LEVY et al. (Biological Psychiatry, Vol. 77, 15 March 2015, pages e27-e29) refers to Clinical and electrophysiological effects of D-serine in a schizophrenia patient positive for anti-NMDA receptor antibodies; LANE et al. (JAMA psychiatry, vol. 70, no. 12, 2013, pages 1267-1275) discloses the use of sodium benzoate for treating schizophrenia; and Akiko Matsuura et al. (Acta Neuropsychiatrica, vol. 27, no. 3, June 2015, pages 159-167) discloses that sodium benzoate can induce antipsychotic effect in the schizophrenia model induced by phencyclidine. However, none of the above publications can effectively prevent or treat anti-NMDAR encephalitis.

Accordingly, there remains a need for the effective prophylaxis or treatment of anti-NMDAR encephalitis.

### SUMMARY

In view of the foregoing, the present disclosure provides a pharmaceutical composition for use in preventing or treating anti-NMDAR encephalitis in a subject in need thereof, wherein the pharmaceutical composition comprises an effective amount of sodium benzoate, and a pharmaceutically acceptable excipient thereof.

In one embodiment of the present disclosure, the anti-NMDAR encephalitis is caused by the hypofunction of NMDAR.

In one embodiment of the present disclosure, the sodium benzoate may be administered to the subject in an amount ranging from 200 mg/day to 2000 mg/day, such as from 400 mg/day to 1800 mg/day, from 450 mg/day to 1500 mg/day, and from 500 mg/day to 1000 mg/day.

In one embodiment of the present application, the sodium benzoate may be administered to the subject in a period ranging from 1 month to 2 years, such as from 4 weeks to 12 months.

### DETAILED DESCRIPTION OF THE EMBODIMENTS

The following examples are used to exemplify the present disclosure. A person of ordinary skill in the art can understand the other advantages of the present disclosure, based on the disclosure of the present specification. The present disclosure can also be implemented or applied as described in different specific examples.

It is further noted that, as used in this specification, the singular forms "a," "an," and "the" include plural referents, unless expressly and unequivocally limited to one referent. The term "or" is used interchangeably with the term "and/or" unless the context clearly indicates otherwise.

Anti-NMDAR encephalitis is an autoimmune disease owing to the passing of autoantibodies across the brain-blood barrier, causing the selective crosslinking and internalization of NMDA receptors in both excitatory and inhibitory hippocampal neurons that result in reduction of NMDAR-mediated synaptic currents and hypofunction of NMDAR.

The present disclosure provides a pharmaceutical composition for use in preventing or treating anti-NMDAR encephalitis in a subject in need thereof, wherein the pharmaceutical composition comprises an effective amount of sodium benzoate, and a pharmaceutically acceptable excipient thereof.

As used herein, the term "treating" or "treatment" refers to administration of an effective amount of sodium benzoate to a subject in need thereof with the purpose of cure, alleviate, relieve, remedy, ameliorate, or prevent the disease, the symptoms thereof, or the predisposition towards it. Such a subject can be identified by a health care professional based on results from any suitable diagnostic method.

In one embodiment of the present disclosure, the subject to be treated with the pharmaceutical composition of the present disclosure suffers from anti-NMDAR encephalitis. The subject diagnosed with anti-NMDAR encephalitis may have headache and nausea, presenting first a psychiatric symptom, followed with neurologic dysfunction. In a further embodiment of the present disclosure, the psychiatric symptom includes agitation, cognitive deterioration, repetitive dissociative memory impairment and those associated with schizophrenia. In another embodiment of the present disclosure, the neurologic dysfunction in the subject diagnosed with anti-NMDAR encephalitis includes epilepsy. In another embodiment, the subject diagnosed with anti-NMDAR encephalitis has anti-NMDAR antibodies identified in the subject's cerebrospinal fluid (CSF) and serum. In another embodiment, the cellular damage in anti-NMDAR encephalitis is caused by autoantibodies, which pass the blood-brain barrier to enter the brain to selectively crosslink and internalize NMDAR in both the excitatory and inhibitory hippocampal neurons. In yet another embodiment, the subject to be treated with the pharmaceutical composition of the present disclosure suffers from anti-NMDAR encephalitis and shows poor response, or is refractory to other therapies.

Benzoic acid occurs naturally in many plants and animals. It is therefore a natural constituent of many foods, including milk products (IPCS 1993). Benzoic acid and sodium benzoate are also legal food additives in USA (Joint FAO/WHO Expert Committee on Food Additives. 1965, 1973), Taiwan (Department of Health), and World Health Organization (IPCS 1993), and are widely used in manufacturing fruit jelly, butter, soy-bean sauce, processed meat, etc.

D-amino acid oxidase (DAAO) is a flavoenzyme of peroxisomes existing in the brain, kidney and liver of mammals, which is responsible for degrading D-serine, D-alanine, and other D-amino acids. In the present disclosure, the benzoic acid salt such as sodium benzoate is administered to inhibit DAAO activity and thereby raise synaptic concentrations of D-serine and other D-amino acids. In the present disclosure, the sodium benzoate is administered to a subject in an effective amount to prevent or treat anti-NMDAR encephalitis through enhancing or activating function of NMDAR. In one embodiment of the present disclosure, the sodium benzoate enhances NMDA function by inhibiting the DAAO activity in a subject with anti-NMDAR encephalitis.

As used herein, the term "effective amount" refers to a therapeutical amount that is sufficient to result in prevention of the development, recurrence, or onset of anti-NMDAR encephalitis and one or more symptoms thereof, to enhance or improve the prophylactic effect of another therapy, reduce the severity, the duration of the disorder, ameliorate one or more symptoms of the disorder, prevent the advancement of anti-NMDAR encephalitis, and/or enhance or improve the therapeutic effect of another therapy.

In some embodiments of the present disclosure, the effective amount of the sodium benzoate may range from 200 mg/day to 2000 mg/day. In an embodiment, a lower limit of the dosage may be 200 mg/day, 225 mg/day, 250 mg/day, 275 mg/day, 300 mg/day, 325 mg/day, 350 mg/day, 400 mg/day, 450 mg/day, 500 mg/day, 550 mg/day, 600 mg/day, 700 mg/day, 750 mg/day, or 800 mg/day and an upper limit of the dosage may be 2000 mg/day, 1800 mg/day, 1500 mg/day, 1200 mg/day, 1000 mg/day, 900 mg/day, 800 mg/day, 750 mg/day, 700 mg/day, or 600 mg/day. For example, the dosage of the sodium benzoate may be 225 mg/day to 2000 mg/day, 250 mg/day to 1800 mg/day, 450 mg/day to 1500 mg/day, 500 mg/day to 1200 mg/day, 500 mg/day to 1000 mg/day, 550 mg/day to 1000 mg/day, 600 mg/day to 800 mg/day, about 1000 mg/day, about 900 mg/day, about 750 mg/day, or about 500 mg/day.

As used herein, when a number or a range is recited, a person having ordinary skill in the art understands that it intends to encompass an appropriate, reasonable range for the particular field related to the disclosure.

By reciting at least 200 mg to 2000 mg, it means that all integer unit amounts within the range are specifically disclosed as part of the disclosure. Thus, 200, 201, 202 ... 250, 251, 252 ... 1000, 1001, 1002 ... 1997, 1998, 1999 and 2000 unit amounts are included as embodiments of the present disclosure.

In some embodiments of the present disclosure, the administration of the sodium benzoate may be conducted, for example, once per day, twice per day, 3 times per day, or 4 times per day. In an embodiment, the administration of the sodium benzoate may be conducted once per day.

In some embodiments of the present disclosure, the sodium benzoate may be administered to the subject in a period sufficient to prevent or treat anti-NMDAR encephalitis. The sufficient period may depend on the species, gender, body weight or age of the subject, the stage, symptom or severity of the disease, and the routes, timing or frequency of the administration. In some embodiments of the present disclosure, the administration of the sodium benzoate is daily over at least 1 month. For example, the period of administration of the sodium benzoate may last for 1, 2, 3, 4, or 6 months, or 1, 2, 3 or 4 years, or even longer, as long as no side effect occurs during the treatment period. In the exemplary embodiments of the present disclosure, the period may be in a range of from 1 month to 2 years. In another embodiment, the period ranges from 4 weeks to 12 months. In yet another embodiment, the administration of the sodium benzoate is daily for 2 months.

In one embodiment of the present disclosure, the sodium benzoate is administered in an oral dosage form. In one embodiment of the present disclosure, the sodium benzoate administered to the subject may be contained in a pharmaceutical composition. The pharmaceutical composition of the present disclosure comprises sodium benzoate and a pharmaceutically acceptable excipient thereof. In an embodiment, the pharmaceutical composition of the present disclosure is formulated in a form suitable for oral administration, and thus the pharmaceutical composition may be administered to the subject by oral delivery. Alternatively, the pharmaceutical composition may be formulated in a form of dry powder, a tablet, a lozenge, a capsule, granule, or a pill. The pharmaceutically acceptable excipient includes, but is not limited to, a filler, a binder, a preservative, a disintegrating agent, a lubricant, a suspending agent, a wetting agent, a solvent, a surfactant, an acid, a flavoring agent, polyethylene glycol (PEG), alkylene glycol, sebacic acid, dimethyl sulfoxide, an alcohol, or any combination thereof.

The pharmaceutical composition of the present disclosure may only comprise the sodium benzoate as an active ingredient for preventing or treating anti-NMDAR encephalitis. In other words, the sodium benzoate serves as the only active ingredient for preventing or treating anti-NMDAR encephalitis in the pharmaceutical composition of the present disclosure. In this embodiment, the present disclosure provides a safe and effective therapy for preventing or treating anti-NMDAR encephalitis by the use of the sodium benzoate alone as the active ingredient.

Many examples have been used to illustrate the present disclosure. The examples below should not be taken as any limit to the scope of the present disclosure.

### EXAMPLE

The present disclosure examined the efficacy and safety of sodium benzoate for the treatment of anti-NMDAR encephalitis.

A previously healthy 17-year-old, male presented with headache and nausea without fever. One week later, he progressively developed agitation, cognitive deterioration with required psychiatric evaluation. The patient was admitted to acute psychiatric ward 3 times due to suspected schizophrenia and repetitive dissociative memory impairment.

The patient was treated with a heavy dose of antipsychotics and electroconvulsive therapy (ECT) but showed very poor response to antipsychotics even at high doses (aripiprazole 20-30 mg, clozapine 300-500 mg, and clozapine 500 mg plus paliperidone 9 mg), and electroconvulsive therapy (thrice a week for three weeks). Anti-NMDAR antibodies were then identified in cerebrospinal fluid (CSF) and serum, and anti-NMDAR encephalitis was confirmed. Brain magnetic resonance imaging was found normal.

The patient was immediately treated with intravenous immunoglobulin (IVIg) and plasmaphoresis after pulse therapy with methylprednisolone. However, no remarkable clinical improvement was noticed after two months of immunotherapy and antipsychotic treatment.

Sodium benzoate was then administered to the patient at a dose of 1000 mg per day for 2 months. The psychotic symptoms including dissociation and agitation were reduced. An obvious clinical improvement was observed on the patient, and he was able to return to a normal life.

The foregoing descriptions of the detailed embodiments are only illustrated to disclose the principle and functions of the present disclosure and do not intend to restrict the scope of the present disclosure. It should be understood to those skilled in the art that all modifications and variations according to the principle of the present disclosure should fall within the scope of the appended claims. It is intended that the specification and examples are considered as exemplary only, with a full scope of the disclosure being indicated by the following claims.
DALMAU, J. (2016) NMDA receptor encephalitis and other antibody-mediated disorders of the synapse: The 2016 Cotzias Lecture. Neurology, 87, 2471-2482.
DALMAU, J., LANCASTER, E., MARTINEZ-HERNANDEZ, E., ROSENFELD, M. R. & BALICE-GORDON, R. (2011) Clinical experience and laboratory investigations in patients with anti-NMDAR encephalitis. Lancet Neurol., 10, 63-74.
LEYPOLDT, F., ARMANGUE, T. & DALMAU, J. (2015) Autoimmune encephalopathies. Annals of the New York Academy of Sciences, 1338, 94-114.
TITULAER, M. J., MCCRACKEN, L., GABILONDO, I., ARMANGUE, T., GLASER, C., IIZUKA, T., HONIG, L. S., BENSELER, S. M., KAWACHI, I., MARTINEZ-HERNANDEZ, E., AGUILAR, E., GRESA-ARRIBAS, N., RYAN-FLORANCE, N., TORRENTS, A., SAIZ, A., ROSENFELD, M. R., BALICE-GORDON, R., GRAUS, F. & DALMAU, J. (2013)
Treatment and prognostic factors for long-term outcome in patients with anti-NMDA receptor encephalitis: an observational cohort study. Lancet Neurol., 12, 157-165.
TSUTSUI, K., KANBAYASHI, T., TAKAKI, M., OMORI, Y., IMAI, Y., NISHINO, S., TANAKA, K. & SHIMIZU, T. (2017) N-methyl-D-aspartate receptor antibody could be a cause of catatonic symptoms in psychiatric patients: case reports and methods for detection. Neuropsychiatr. Dis. Treat., 13, 339-345.

## Claims

1. A pharmaceutical composition for use in preventing or treating anti-N-methyl-D-aspartate receptor (NMDAR) encephalitis in a subject in need thereof,
wherein the pharmaceutical composition comprises an effective amount of sodium benzoate, and a pharmaceutically acceptable excipient thereof.

2. The pharmaceutical composition for use according to claim 1, wherein the anti-NMDAR encephalitis is caused by hypofunction of NMDAR.

3. The pharmaceutical composition for use according to claim 1, wherein the sodium benzoate is administered to the subject in an amount ranging from 200 mg/day to 2000 mg/day.

4. The pharmaceutical composition for use according to claim 1, wherein the sodium benzoate is administered to the subject in an amount ranging from 400 mg/day to 1800 mg/day.

5. The pharmaceutical composition for use according to claim 1, wherein the sodium benzoate is administered to the subject in an amount ranging from 450 mg/day to 1500 mg/day.

6. The pharmaceutical composition for use according to claim 1, wherein the sodium benzoate is administered to the subject in an amount ranging from 500 mg/day to 1000 mg/day.

7. The pharmaceutical composition for use according to claim 1, wherein the sodium benzoate is administered to the subject in a period ranging from 1 month to 2 years.

8. The pharmaceutical composition for use according to claim 1, wherein the sodium benzoate is administered to the subject in a period ranging from 4 weeks to 12 months.

## Patentansprüche

1. Pharmazeutische Zusammensetzung zur Verwendung bei der Vorbeugung oder Behandlung einer Anti-N-Methyl-D-Aspartat-Rezeptor(NMDAR)-Enzephalitis bei einem Probanden, der diese benötigt,
wobei die pharmazeutische Zusammensetzung eine effektive Menge an Natriumbenzoat und einen pharmazeutisch akzeptablen Hilfsstoff davon umfasst.

2. Pharmazeutische Zusammensetzung zur Verwendung nach Anspruch 1, wobei die Anti-NMDAR-Enzephalitis durch eine Hypofunktion des NMDAR verursacht wird.

3. Pharmazeutische Zusammensetzung zur Verwendung nach Anspruch 1, wobei dem Probanden das Natriumbenzoat in einer Menge von 200 mg/Tag bis 2000 mg/Tag verabreicht wird.

4. Pharmazeutische Zusammensetzung zur Verwendung nach Anspruch 1, wobei dem Probanden das Natriumbenzoat in einer Menge von 400 mg/Tag bis 1800 mg/Tag verabreicht wird.

5. Pharmazeutische Zusammensetzung zur Verwendung nach Anspruch 1, wobei dem Probanden das Natriumbenzoat in einer Menge von 450 mg/Tag bis 1500 mg/Tag verabreicht wird.

6. Pharmazeutische Zusammensetzung zur Verwendung nach Anspruch 1, wobei dem Probanden das Natriumbenzoat in einer Menge von 500 mg/Tag bis 1000 mg/Tag verabreicht wird.

7. Pharmazeutische Zusammensetzung zur Verwendung nach Anspruch 1, wobei dem Probanden das Natriumbenzoat über einen Zeitraum von 1 Monat bis 2 Jahren verabreicht wird.

8. Pharmazeutische Zusammensetzung zur Verwendung nach Anspruch 1, wobei dem Probanden das Natriumbenzoat über einen Zeitraum von 4 Wochen bis 12 Monaten verabreicht wird.

## Revendications

1. Une composition pharmaceutique destinée à prévenir ou traiter l'encéphalite anti-récepteur N-méthyl-D-aspartate (NMDAR) chez un sujet en a besoin,
ladite composition pharmaceutique comprenant une quantité efficace de benzoate de sodium, ainsi qu'un excipient pharmaceutiquement acceptable.

2. La composition pharmaceutique selon la revendication 1, dans laquelle l'encéphalite anti- NMDAR est causée par une hypofonction du NMDAR.

3. La composition pharmaceutique selon la revendication 1, dans laquelle le benzoate de sodium est administré au sujet à une dose comprise entre 200 mg/jour et 2000 mg/jour.

4. La composition pharmaceutique selon la revendication 1, dans laquelle le benzoate de sodium est administré au sujet à une dose comprise entre 400 mg/jour et 1800 mg/jour.

5. La composition pharmaceutique selon la revendication 1, dans laquelle le benzoate de sodium est administré au sujet à une dose comprise entre 450 mg/jour et 1500 mg/jour.

6. La composition pharmaceutique selon la revendication 1, dans laquelle le benzoate de sodium est administré au sujet à une dose comprise entre 500 mg/jour et 1000 mg/jour.

7. La composition pharmaceutique selon la revendication 1, dans laquelle le benzoate de sodium est administré au sujet sur une période comprise entre 1 mois et 2 ans.

8. La composition pharmaceutique selon la revendication 1, dans laquelle le benzoate de sodium est administré au sujet sur une période comprise entre 4 semaines et 12 mois.
